# EUROPÄISCHE PATENTSCHRIFT

(11) **EP 1 749 505 B1**
(45) Veröffentlichungstag und Bekanntmachung des Hinweises auf die Patenterteilung: **20.02.2008**
(21) Anmeldenummer: 06010274.6
(22) Anmeldetag: 18.05.2006
(51) Int. Cl.: A61F 2/38

(54) **Gelenkprothese mit Elastomer- oder Federelement**
Joint prosthesis with elastomeric or spring element
Prothèse articulaire avec un élément élastomère ou de ressort

(30) Priorität: 01.08.2005 DE 102005036080; 08.09.2005 DE 102005042773
(43) Veröffentlichungstag der Anmeldung: 07.02.2007
(73) Patentinhaber: Mathys AG Bettlach, 2544 Bettlach (CH)
(72) Erfinder: Delfosse, Daniel, Dr., 3306 Jegensdorf (CH); Fankhauser, Christoph, 4500 Solothurn (CH); Baumgartner, Daniel, 4702 Olnsingen (CH)
(74) Vertreter: Körfer, Thomas

(56) Entgegenhaltungen:
- WO-A-01/97719
- DE-A1- 3 840 474
- DE-A1- 10 012 059
- DE-A1- 10 118 947
- FR-A- 2 734 709
- US-A- 6 139 580

## Beschreibung

Die Erfindung betrifft eine Gelenkprothese zum Ausbilden einer Gelenkverbindung zwischen einem Paar menschlicher oder tierischer Knochen. Die Erfindung betrifft insbesondere eine Kniegelenkprothese zum Ausbilden einer Gelenkverbindung zwischen einem ersten Knochen (Tibia) und zumindest einem zweiten Knochen (Femur).

Aus der DE 101 09 804 A1 ist eine Gelenkprothese, insbesondere eine Kniegelenkprothese, zum Ausbilden einer Gelenkverbindung zwischen einem ersten Knochen und zumindest einem zweiten Knochen mit den gattungsbildenden Merkmalen des Anspruchs 1 bekannt. Die Gelenkprothese weist eine erste Komponente, die zumindest mittelbar mit dem ersten Knochen verbunden ist, zumindest eine zweite Komponente, auf der sich der zweite Knochen zumindest mittelbar abstützt und die auf zumindest einer Lagerfläche der ersten Komponente bewegbar gelagert ist, und ein Verbindungselement auf, das die erste Komponente mit der zweiten Komponente verbindet. Dabei umfaßt das Verbindungselement ein elastisch verformbares Element, das eine Auslenkung der ersten Komponente relativ zu der zweiten Komponente aus einer Ausgangsstellung ermöglicht. Ferner erzeugt bei einer Auslenkung der ersten Komponente das elastisch verformbare Element eine auf die erste Komponente einwirkende Rückstellkraft zur Rückstellung der ersten Komponente in die Ausgangsstellung.

Die aus der DE 101 09 804 A1 bekannte Gelenkprothese hat den Nachteil, daß das Verbindungselement kompliziert aufgebaut und dadurch anfällig für Fehlfunktionen ist. Weiterhin sind durch die geometrischen Gegebenheiten erhebliche Kräfte und Momente zu erwarten, die zu einer hohen Verschleißanfälligkeit führen, was ebenfalls zu einer hohen Revisionsrate führen kann.

Weiterhin ist aus der US 6,770,098 B1 eine Kniegelenkprothese bekannt, welche einen Tibiateil mit einer flachen tibialen Anlagefläche sowie einen Lagerkörper umfaßt, welcher darauf in anterio-posteriorer Richtung verschieblich ist und zwei konkave Lagerschalen aufweist, in welchen ein Femurgelenkteil beweglich gelagert werden kann. Eine Rotationsführung ermöglicht Drehbewegungen des Lagerkörpers auf der tibialen Anlagefläche um eine Rotationsachse, die senkrecht zu der Anlagefläche steht. Die Rotationsführung umfaßt dabei einen Führungsstab, der in dem Lagerkörper beweglich angebracht ist, so daß der Tibiateil in medio-lateraler Richtung und/oder in Drehung geführt ist.

Nachteilig an der aus der US 6,770,098 B1 bekannten Gelenkprothese ist dabei insbesondere, daß der Führungsstab aus einem sehr steifen Werkstoff hergestellt ist, welches bei gegebener Last wenig bis keine Dehnung erfährt. Dadurch ist der Bewegungsspielraum eingeschränkt, die Ausgleichsbewegungen werden unergonomisch und abrupt gestoppt und führen dadurch zu erhöhtem Verschleiß und schlimmstenfalls zu der Notwendigkeit einer baldigen Nach- oder Neuversorgung des Patienten.

Die DE 101 18 947 A1 offenbart ein Kniegelenk-Endoprothesensysstem, das aus einer Femurkomponente, einem Gleitlager, einer Führungseinheit sowie einem Tibiaplateau besteht. Die Verbindung von dem Gleitlager zum Tibiaplateau wird mit Hilfe der Führungseinheit mit Hilfe eines Feder-/Dämpfungsmechanismus stabilisiert. Der Feder-/Dämpfungsmechanismus ist derartig ausgebildet, dass Schraubenfedern in eine Senkung des Tibiaplateaus, welches zur Stabilitätserhöhung einen zentralen Konus aufweist, eingreifen.

Die FR-A-2 734 709 offenbart eine Prothese mit einem femoralen Abschnitt, einem tibialen Abschnitt, einem Schlitten und einer Rolle. Die Form des Kontaktbereichs zwischen dem femoralen Abschnitt und dem Rest der Prothese ist spiralförmig mit variablem Radius. Der Kontaktbereich kann durch geradlinige Abschnitte gebildet werden, welche ausgerichtet, jedoch getrennt durch einen Kreisbogen sind, welcher eine Konkavität oder eine Konvexität in dem femoralen Abschnitt bildet. Der tibiale Abschnitt nimmt ein stoßdämpfendes Material auf.

Der Erfindung liegt demnach die Aufgabe zugrunde, eine Gelenkprothese bereitzustellen, bei der die Bewegung der ersten Komponente relativ zu der zweiten Komponente, insbesondere eine Bewegung in anterio-posteriorer Richtung und/oder medio-lateraler Richtung verbessert ist, und bei der insbesondere eine Anpassung an den natürlichen Bewegungsablauf eines Kniegelenks erreicht wird, selbst wenn eine Beeinträchtigung des die Gelenkprothese unterstützenden Bandapparats vorliegt.

Die Aufgabe wird durch eine Gelenkprothese mit den Merkmalen des Anspruchs 1 gelöst. Vorteilhafte Weiterbildungen der Erfindung sind durch die in den Unteransprüchen angegebenen Maßnahmen möglich.

Die erfindungsgemäße Gelenkprothese hat den Vorteil, daß eine Auslenkung der ersten Komponente relativ zu der zweiten Komponente aus einer Ausgangsstellung über das elastisch verformbare Element erfolgt, welches aus einem dämpfenden Element oder einem Federelement besteht und welches weiterhin in der ersten Komponente der Gelenkprothese angeordnet ist.

Dadurch wird auch bei einem geschwächten oder beschädigten Bandapparat eine Anpassung des Bewegungsablaufs mittels der Gelenkprothese an den natürlichen Bewegungsablauf erreicht. Außerdem ist sichergestellt, daß im entspannten Zustand die Gelenkprothese in ihre Ausgangsstellung zurückgestellt wird. Dadurch kann auch bei Patienten mit gestörtem bzw. geschwächtem Bandapparat eine natürliche Kinematik der Gelenkprothese erzielt werden. Die Bandstabilität kann dabei durch einstellbare Federkonstanten des elastisch verformbaren Elements oder Federelements ergänzt, kompensiert bzw. optimiert werden, wodurch eine Anpassung an Gewicht, Aktivitätslevel und den bestehenden Bandapparat des Patienten möglich ist. Die Funktion des beim Totalen Knieersatz geopferte vordere Kreuzband kann beispielsweise durch dieses Dämpfungs- und/oder Federelement zumindest teilweise ersetzt oder kompensiert werden.

Außerdem kann durch das elastisch verformbare Element eine Fehlstellung des sich zumindest mittelbar auf der zweiten Komponente abstützenden zweiten Knochens relativ zu den Lagerflächen der zweiten Komponente ausgeglichen werden. Eine solche Fehlstellung kann durch eine Fehlstellung des ersten Knochens relativ zu dem zweiten Knochen oder aufgrund der bei der Operation bestehenden Toleranz gegeben sein. Üblicherweise liegt die axiale Fehlstellung im Bereich von einigen Millimetern. Diese fehlerhafte Gelenkausrichtung führt zu höheren Scherkräften und einem stärkeren seitlichen Abrieb der Gelenkprothese und zu einer asymmetrischen Belastung der Bandstrukturen. Sie kann zudem zu einem eingeschränkten Bewegungsumfang führen. Diese fehlerhafte Gelenkausrichtung wird durch die erfindungsgemäße Gelenkprothese mittels des elastisch verformbaren Elements aufgehoben bzw. kompensiert.

Die Erfindung wird nachfolgend anhand von Ausführungsbeispielen unter Bezugnahme auf die Zeichnungen näher beschrieben. In den Zeichnungen zeigen:
- Fig. 1A: ein Ausführungsbeispiel eines Verbindungselements für eine Gelenkprothese gemäß dem Stand der Technik in einem sagittalen Schnitt;
- Fig. 1B: das in Fig. 1A gezeigte Verbindungselement in einem frontalen Schnitt;
- Fig. 1C: eine Aufsicht in longitudinaler Richtung auf das in den Fig. 1A und 1B seitlich dargestellte Ausführungsbeispiel gemäß dem Stand der Technik;
- Fig. 2A-F: Ausführungsbeispiele von tibialen Komponenten für Kniegelenk-Endoprothesen in seitlichen teilweise geschnittenen Ansichten;
- Fig. 3A-H: einige der in Fig. 2 dargestellten Beispiele der elastischen Elemente tibialer Komponenten in perspektivischer Ansicht.

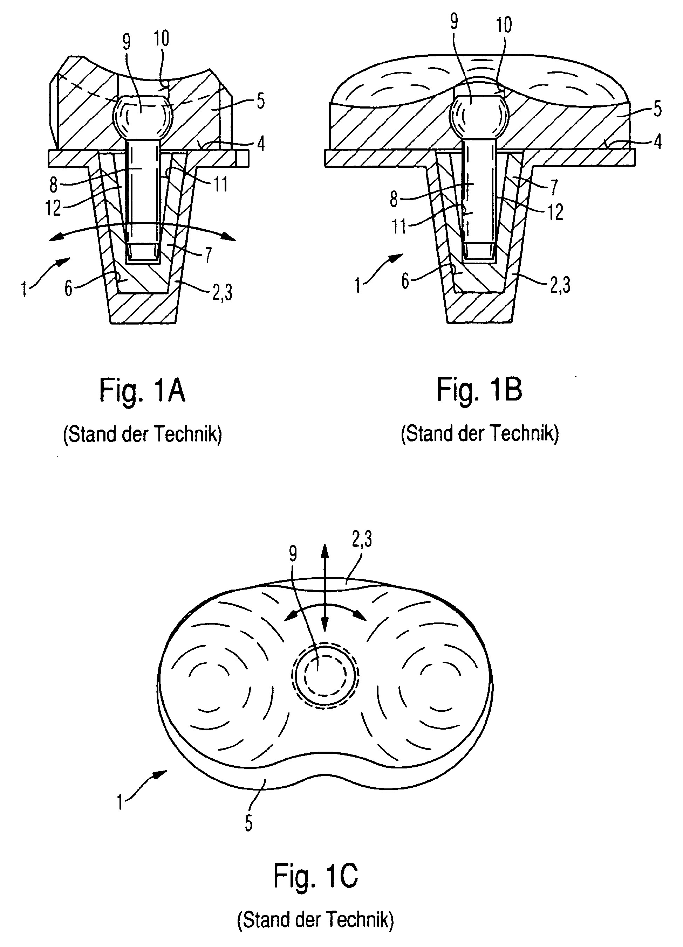

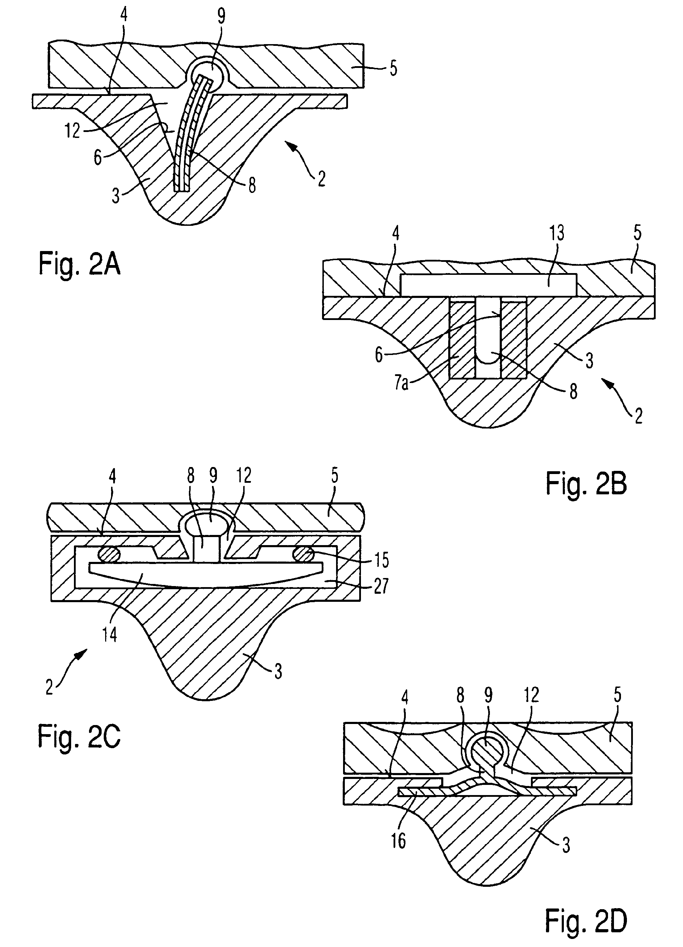

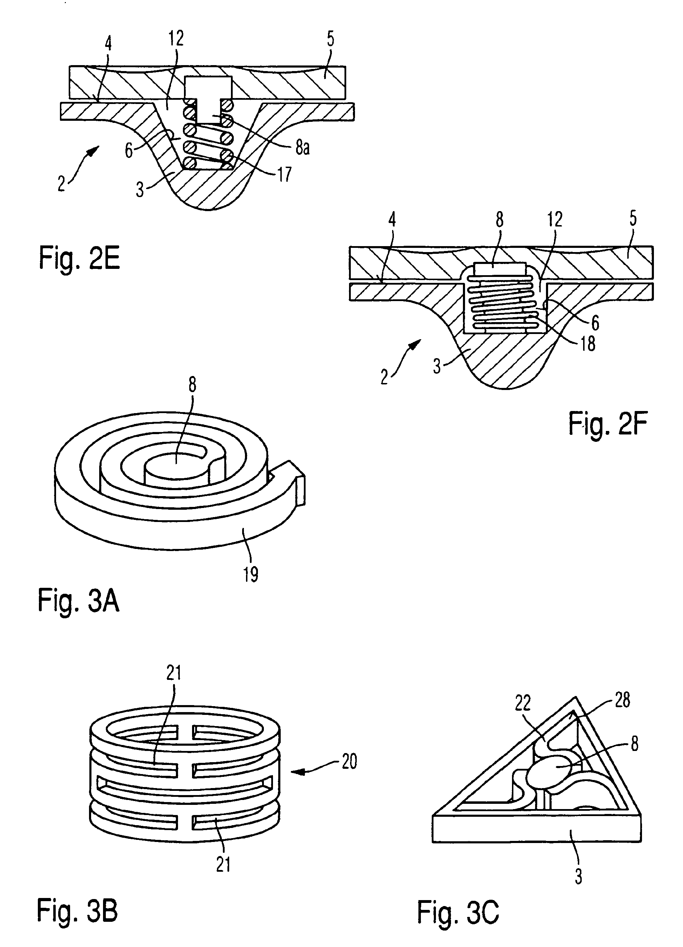

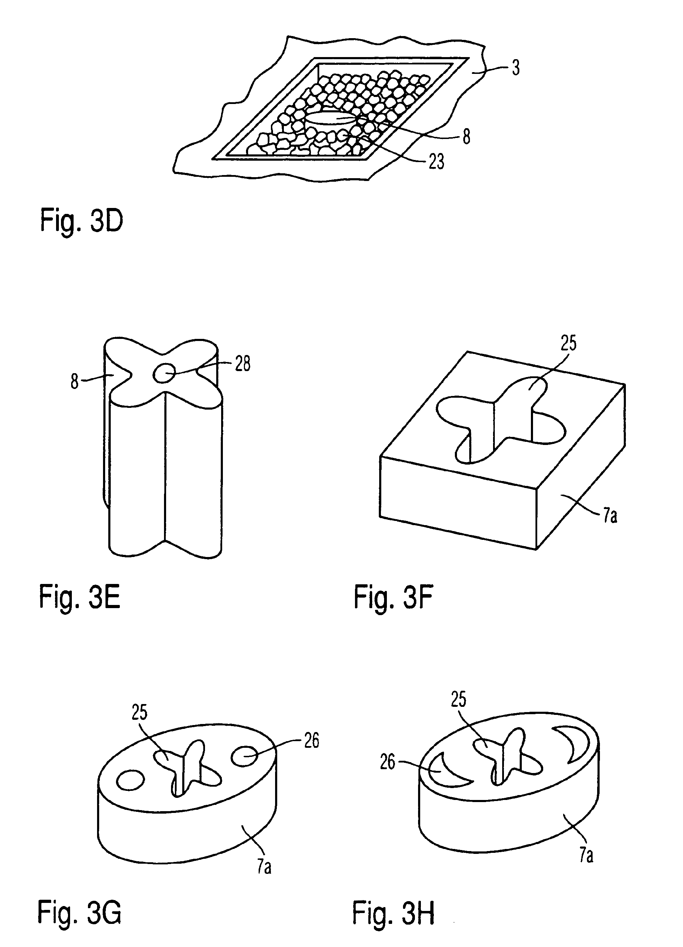

Die Fig. 1A bis 1C zeigen in zwei frontal geschnittenen Ansichten bzw. in einer Aufsicht nach distal ein Ausführungsbeispiel der tibialen Komponente 2 einer Kniegelenk-Endoprothese 1 gemäß dem Stand der Technik zur besseren Verständlichkeit der im Folgenden unter Bezugnahme auf die Fig. 2 und 3 erläuterten erfindungsgemäßen Maßnahmen.

Die tibiale Komponente 2 der Kniegelenk-Endoprothese 1 weist einen Grundkörper 3 mit einer proximalen Anlagefläche 4 auf, an welcher eine Lagerschale 5 verschieblich angeordnet ist. Die Lagerschale 5 ist proximal geeignet ausgeformt, um eine Gleitfläche für die Femurkondylen zu bilden.

Die Anlagefläche 4 und die Lagerschale 5 sind in ihrer Form zueinander hoch kongruent. In dem Grundkörper 3 ist eine Ausnehmung 6 ausgebildet, in welcher ein Einsatz 7 angeordnet ist. Der Einsatz 7 liegt formschlüssig an einer Wandung der Ausnehmung 6 an und ist in dieser fixiert.

In dem Einsatz 7 ist ein Führungsstab 8 angeordnet, welcher sich aus dem Grundkörper 3 in die Lagerschale 5 erstreckt. An einem proximalen Ende des Führungsstabs 8 ist ein Verbindungskörper 9 ausgebildet, der in Eingriff mit einer Ausnehmung 10 der Lagerschale 5 steht und dabei beispielsweise ein Kugelgelenk bildet.

Der Einsatz 7 für den Führungsstab 8 ist dabei so ausgebildet, daß der Führungsstab 8 mit der damit kraftschlüssig verbundenen Lagerschale 5 in mehreren Freiheitsgraden beweglich ist. Die möglichen Bewegungsrichtungen sind in Fig. 1C durch Pfeile gekennzeichnet. Die Lagerschale 5 ist dabei in anterio-posteriorer Richtung sowie rotatorisch beweglich, nicht jedoch in medial-lateraler Richtung, was zu einer unerwünschten Instabilität des Kniegelenks führen würde. Die rotatorische und anterio-posteriore Beweglichkeit dagegen ermöglicht dem prothetisch versorgten Kniegelenk eine verbesserte physiologische Beweglichkeit.

Die Begrenzung der möglichen Freiheitsgrade wird dabei durch die Ausformung einer Ausnehmung 11 in dem Einsatz 7 erzielt, in welcher sich, wie aus den Fig. 1A und 1B in zwei zueinander senkrechten Längsschnitten ersichtlich, der Führungsstab 8 bewegt. In Fig. 1A ist ein Spalt 12 so weit, daß eine erhöhte Beweglichkeit des Führungsstabs 8 möglich ist, während in der Richtung senkrecht dazu der Spalt 12 so eng ist, daß eine Führung des Führungsstabs 8 ohne weiteren Freiheitsgrad erzielt wird, wie aus Fig. 1B ersichtlich.

Nachteilig an dem dargestellten Ausführungsbeispiel einer Kniegelenk-Endoprothese 1 gemäß dem Stand der Technik ist dabei insbesondere, daß die Beweglichkeit des Führungsstabes 8 durch die Form der Ausnehmung 11 bzw. durch das Material des Führungsstabes 8 eingeschränkt bzw. physiologisch ungünstig ist. Der Bewegungsspielraum, der durch die Form des Einsatzes 7 vorgegeben ist, wird bei Anschlag des Führungsstabes 8 am Rand abrupt begrenzt, was einerseits Schädigungen im Bereich der Kniegelenkprothese 1 und weiterhin einen unergonomischen oder sogar unangenehmen Bewegungsverlauf mit nachfolgenden Haltungsfehlern durch Schonhaltung etc. beim Patienten begünstigt.

Bei anderen Ausführungsformen gemäß dem Stand der Technik ist weiterhin bekannt, in der Kniegelenk-Endoprothese 1 einen elastischen Rückstellmechanismus vorzusehen, welcher in einer femuralen Komponente der Kniegelenk-Endoprothese 1 intergriert ist. Diese Ausführungsform ist insbesondere deshalb nachteilig, weil der Rückstellmechanismus aufgrund der langen Hebelwege anfällig auf Beschädigungen ist und somit teuer in der Herstellung wird, da keine Zugeständnisse in Bezug auf die Haltbarkeit und Zuverlässigkeit der Kniegelenk-Endoprothese 1 gemacht werden können.

Erfindungsgemäß wird daher vorgeschlagen, die Materialien bzw. die Form des Führungsstabes 8 so zu wählen, daß ein gedämpfter physiologischer Bewegungsverlauf bei guten Führungseigenschaften und einfachem Aufbau mit Integration in der tibialen Komponente 2 der Kniegelenk-Endoprothese 1 ermöglicht wird. Ausführungsbeispiele entsprechend ausgeformter tibialer Komponenten 2 für Kniegelenk-Endoprothesen 1 sind in den Fig. 2A bis 2F, 3A bis 3H dargestellt. Übereinstimmende Bauteile sind dabei in allen Figuren mit übereinstimmenden Bezugszeichen versehen.

Allen in Fig. 2 dargestellten Ausführungsbeispielen einer tibialen Komponente 2 einer Kniegelenk-Endoprothese 1 ist dabei gemeinsam, daß die Bauteile, welche für die Führung der Lagerschale 5 verantwortlich sind, in der tibialen Komponente angeordnet sind. Weiterhin sind die die Führung der Lagerschale 5 bestimmenden Bauteile aus elastischen Materialien gestaltet.

Fig. 2A zeigt in gleicher Darstellung wie Fig. 1A ein erstes Ausführungsbeispiel einer erfindungsgemäßen tibialen Komponente 2 einer Kniegelenk-Endoprothese 1.

Neben einer ansonsten im Wesentlichen identischen Ausführung weist das erste Ausführungsbeispiel einen Führungsstab 8 auf, welcher aus einem elastischen Material und/oder hohlzylindrisch hergestellt ist und mit seinem Verbindungskörper 9 in die Lagerschale 5 eingreift. Die durch die elastischen Eigenschaften des Führungsstabes 8 ermöglichte Beweglichkeit des Kniegelenks ist für den Patienten erheblich angenehmer und für die Endoprothese 1 verschleißärmer als die herkömmlichen Führungsstäbe 8 aus einem vollzylindrischen Hartplastikmaterial.

Fig. 2B zeigt ein Beispiel einer tibialen Komponente 2. Hier ist der Einsatz 7 in Form einer elastischen Hülse 7a ausgebildet, in welcher der Führungsstab 8 eingreift. Dieser ist an einer in der Lagerschale 5 verankerten Platte 13 entweder einstückig oder fest verbunden ausgebildet, welche die Eigenschaften des kugelförmigen Körpers 9 des weiter oben beschriebenen ersten Ausführungsbeispiels übernimmt.

Die Hülse 7a ist ebenfalls aus einem elastischen Material hergestellt und zylindrisch ausgebildet. Die Beweglichkeit des Führungsstabes 8 wird hierbei durch die Elastizität der Hülse 7a gewährleistet. Beispielsweise durch die Einarbeitung von Versteifungselementen können dann die Freiheitsgrade der Bewegung auf anterio-posterior sowie rotatorisch eingeschränkt werden.

In Fig. 2C ist ein Beispiel dargestellt, welches einen Führungsstab 8 aufweist, der an seinem distalen Ende einen kugelsegmentförmigen oder walzensegmentförmigen Körper 14 aufweist, welcher kipp- und drehbar in einer Ausnehmung 27 der tibialen Komponente 2 gelagert ist. Der Körper 14 stützt sich dabei über ein ringförmiges Dämpfungselement 15 ab, welches einerseits für einen begrenzten Bewegungsspielraum sorgt und andererseits ein zu starkes Verdrehen des Körpers 14 vermeidet. Durch eine geeignete Ausformung des Körpers 14 bzw. der tibialen Komponente 2 kann die Beweglichkeit des mit dem Körper 14 verbundenen Führungsstabes 8 auf die gewünschte anterio-posteriore Bewegungsrichtung und einen rotatorischen Freiheitsgrad eingegrenzt werden.

Fig. 2D zeigt ein zweites Ausführungsbeispiel einer erfindungsgemäß ausgestalteten tibialen Komponente 2, welche zur Verankerung in dem Grundkörper 3 eine Tellerfeder 16 aufweist, an welcher der Führungsstab 8 mit dem kugelförmigen Körper 9 ausgebildet ist. Die Elastizität der Tellerfeder 16 sorgt für den Bewegungsspielraum, welcher durch eine Anpassung der Form der Tellerfeder 15 in die gewünschten Freiheitsgrade eingeschränkt werden kann. Hierbei wird die Elastizität in anterio-posteriorer Richtung durch eine Elastizität in distal-proximaler Richtung ergänzt. Die Tellerfeder kann dabei kreisförmig, sternförmig, oval oder in einer beliebigen anderen Form ausgebildet sein, um die Freiheitsgrade der Bewegung festzulegen.

Ausgehend von der Tellerfeder 16 gemäß dem vorstehend beschriebenen zweiten Ausführungsbeispiel weist das in Fig. 2E dargestellte dritte Ausführungsbeispiel eine Schraubenfeder 16 auf, welche auch teilweise die Funktion des Führungsstabes 8 übernimmt. Dieser ist nur noch in Form eines Stummels 8a ausgebildet, welcher sich in Richtung distal in die Schraubenfeder 17 hinein erstreckt.

Eine weitere Möglichkeit besteht gemäß Fig. 2F darin, den Führungsstab 8 noch weiter zu verkürzen bzw. ganz entfallen zu lassen und statt dessen gemäß einem sechsten Ausführungsbeispiel einen Faltenbalg 18 einzusetzen. Durch eine geeignete Wahl der Materialien bzw. der Steifigkeit des Faltenbalgs 18 können auch hier der Bewegungsspielraum und die Freiheitsgrade gemäß den Vorgaben angepaßt werden.

Die Fig. 3A bis 3H zeigen stark schematisierte perspektivische Ansichten von bevorzugten Lagerungsmöglichkeiten des Führungsstabes 8 gemäß dem vorstehend insbesondere unter Bezugnahme auf die Fig. 2B beschriebenen Beispiel einer erfindungsgemäßen tibialen Komponente 2 für eine Kniegelenk-Endoprothese 1.

Ist die Hülse 7a gemäß Fig. 2B beispielsweise, wie in Fig. 3A dargestellt, in Form einer spiralig mehrschichtig gewundenen Kunststofflage 19 ausgebildet, welche mit dem zentral darin angeordneten Führungsstab 8 entweder einstückig ausgebildet oder an diesem fixiert ist, ist eine elastische Lagerung des Führungsstabes 8 gewährleistet. Die Deformation der elastischen Hülse, wie beispielsweise in Fig. 3A dargestellt, kann durch die Wahl des Werkstoffes und des Design der Hülse in die gewünschte Richtung eingestellt werden, was entsprechend eine Auslenkung der Lagerschale in Fig. 2A - 2F zur Folge hat. Die Definition der Freiheitsgrade der Lagerschale 5 kann somit durch eine geeignete Ausformung des Grundkörpers 3 der tibialen Komponente 2 im Bereich der Ausnehmung 6 erfolgen.

Auch eine Rohrfeder 20, wie aus Fig. 3B hervorgeht, ist als Ausführungsform für die Hülse 7a vorteilhaft. Durch eine geschickte Wahl der Ausnehmungen 21, welche die Elastizität und das Biegeverhalten der Rohrfeder 20 beeinflussen, können auch hier die Bewegungsrichtungen in translatorischen als auch in rotatorischen Freiheitsgraden den Anforderungen angepaßt werden.

Fig. 3C zeigt eine weitere Möglichkeit der Lagerung des Führungsstabes 8 durch Blattfedern 22. Im Beispiel sind drei Blattfedern 22 dargestellt, welche sich in der entsprechend geformten Ausnehmung 6 des Grundkörpers 3 der tibialen Komponente 2 abstützen. Die Freiheitsgrade können hierbei durch eine geeignete Wahl der Anzahl von Blattfedern 22 festgelegt werden.

In Fig. 3D ist eine weitere vorteilhafte Variante zur Lagerung des Führungsstabes 8 in dem Grundkörper 3 der tibialen Komponente 2 dargestellt. Hierbei ist der Führungsstab 8 in kugelförmigem Schüttgut 23, ein Füllmaterial oder Granulat, gelagert, welches beispielsweise aus Elastomerkügelchen besteht.

Neben der Beeinflussung der Freiheitsgrade der Beweglichkeit der Kniegelenkprothese 1 durch die Lagerung des Führungsstabes 8 ist es weiterhin möglich, die Bewegungsrichtungen der Lagerschale 5 relativ zum Grundkörper 4 der tibialen Komponente 2 durch eine geeignete Querschnittsform des Führungsstabes 8 bzw. der den Führungsstab 8 führenden Hülse 7a zu beeinflussen.

Die Fig. 3E bis 3H zeigen vorteilhafte Ausführungsformen für Hülsen 7a für Führungsstäbe 8 für tibiale Komponenten 2 für Kniegelenkprothesen 1 in einer stark schematisierten Darstellung.

Die Ausformung der Hülse 7a bzw. des Führungsstabes 8 muß dabei mehreren Kriterien genügen: einerseits ist eine erhöhte Beweglichkeit in anterio-posteriorer Richtung erwünscht, während die Beweglichkeit in medial-lateraler Richtung nur geringfügig sein darf. Andererseits muß auch die Möglichkeit zur torsionalen Verdrehung des Führungsstabes 8 in der Hülse 7a bestehen, um den rotatorischen Freiheitsgrad der Lagerschale 5 auf der Anlagefläche 4 der tibialen Komponente 2 zu erschließen.

Diese Voraussetzungen können beispielsweise durch eine mit einem entsprechenden Innenquerschnitt ausgestattete Hülse 7a mit einem in der Form korrespondierenden Führungsstab 8 erzielt werden. Eine derartige korrespondierende Form von Außen- und Innenquerschnitt von Führungsstab 8 und Hülse 7a ist stark schematisiert in Fig. 3E und 3F dargestellt.

Fig. 3E zeigt dabei einen Führungsstab 8, welcher einen ungefähr kreuzförmigen Querschnitt aufweist. Zur Anpassung der Elastizität des Führungsstabes 8 kann beispielsweise ein Balken des Kreuzes länger sein als der andere, so daß in einer Biegerichtung eine andere Biegesteifigkeit als in der Richtung senkrecht dazu erzielt wird. Zur Versteifung aus Stabilitätsgründen kann in dem Führungsstab 8 auch ein beispielsweise metallischer Versteifungsstab 28 o.ä. eingearbeitet sein.

In Fig. 3F ist die mit dem Führungsstab 8 korrespondierende Hülse 7a dargestellt, welche eine zentrale Ausnehmung 25 in Kreuzform aufweist. Durch eine entsprechende Gestaltung der Kreuzform, beispielsweise ebenfalls durch die Verlängerung der Ausnehmung 25 in einer oder mehreren Richtungen, kann die Elastizität weiter angepaßt werden. Der Außenquerschnitt der Hülse 7a kann dabei beliebige Formen annehmen, welche wiederum der Querschnittsform der Ausnehmung 6 des Grundkörpers 3 der tibialen Komponente 2 der Gelenkprothese 2 angepaßt ist. Im Ausführungsbeispiel ist dies eine rechteckige Form, es sind jedoch auch runde oder ovale Formen möglich, wie in den folgenden Ausführungsbeispielen dargestellt.

Besonders vorteilhaft für die Hülse 7a sind ovale Querschnittsformen, wie sie in den Fig. 3G und 3H dargestellt sind. Hierbei ist die Elastizität in einfacher und kostengünstiger Weise durch die Einbringung weiterer, beispielsweise runder, ovaler oder sichelförmiger Ausnehmungen 26 in das Material der Hülse 7a in verschiedenen Bewegungsrichtungen beeinflußbar.

Die Hülse 7a bzw. der Führungsstab 8 sind dabei vorzugsweise aus Materialien hergestellt, welche einerseits biokompatibel, andererseits elastisch genug sind, die gewünschten Eigenschaften hinsichtlich Elastizität, Torsionsfähigkeit und Stabilität zu erfüllen.

Der in Fig. 2A und 2B dargestellte Führungsstab 8 bzw. der Körper 14 in Fig. 2C, die Tellerfeder 16 gemäß Fig. 2D, die Schraubenfeder 17 gemäß Fig. 2E und der Federbalg 18 gemäß Fig. 2F sind dabei vorzugsweise aus Metall gefertigt, während die Hülse 7a in Fig. 2B und der Dämpfungsring 15 gemäß Fig. 2C aus einem biokompatiblem Kunststoff gefertigt sind. Die Lagerschale 5 ist jeweils aus einem Polymer wie beispielsweise Polyethylen hergestellt, welches einen guten Gleitpartner für die Gelenkprothese 1 darstellt.

Die Erfindung ist nicht auf die beschriebenen Ausführungsbeispiele beschränkt, sondern auch für andere Bauformen von Kniegelenkprothesen anwendbar. Alle Merkmale der Erfindung sind beliebig miteinander kombinierbar.

## Patentansprüche

1. Gelenkprothese (1), insbesondere Kniegelenkprothese (1), zum Ausbilden einer Gelenkverbindung zwischen einem ersten Knochen und zumindest einem zweiten Knochen mit einer ersten, insbesondere tibialen Komponente (2), die zumindest mittelbar mit dem ersten Knochen verbunden ist, zumindest einer zweiten Komponente, auf der sich der zweite Knochen zumindest mittelbar abstützt und die auf zumindest einer Lagerschale (5) der ersten, tibialen Komponente (2) bewegbar gelagert ist,
einem Führungselement (8), durch welches eine bewegliche Verbindung zwischen der ersten, tibialen Komponente (2) und der Lagerschale (5) gebildet ist, und
einem elastisch verformbaren Element, das eine Auslenkung der Lagerschale (5) relativ zu der ersten Komponente (2) aus einer Ausgangsstellung sowie eine Rückstellung in die Ausgangsstellung ermöglicht, und welches in der ersten Komponente (2) der Gelenkprothese (1) angeordnet ist,
**dadurch gekennzeichnet,**
**dass** das Führungselement (8) als Führungsstab ausgebildet ist, welcher an einem proximalen Ende einen Verbindungskörper (9) aufweist, der in Eingriff mit einer Ausnehmung (10) der Lagerschale (5) steht, wobei der Führungsstab aus einem elastischen Material hergestellt ist.

2. Gelenkprothese nach Anspruch 1,
**dadurch gekennzeichnet,**
**dass** das elastisch verformbare Element in anterioposteriorer Richtung leichter verformbar ist als in medial-lateraler Richtung.

3. Gelenkprothese nach Anspruch 1 oder 2,
**dadurch gekennzeichnet,**
**dass** das elastisch verformbare Element in Form eines Elastomerelements (7a; 15; 19; 23) ausgebildet ist.

4. Gelenkprothese nach einem der Ansprüche 1 bis 3,
**dadurch gekennzeichnet,**
**dass** das elastisch verformbare Element aus einem Polymer, insbesondere aus Polyethylen, besteht.

5. Gelenkprothese nach einem der Ansprüche 1 bis 4,
**dadurch gekennzeichnet,**
**dass** das elastische Element in Form einer Hülse (7a) ausgebildet ist.

6. Gelenkprothese nach Anspruch 5,
**dadurch gekennzeichnet,**
**dass** die Hülse (7a) in einer Ausnehmung (6) eines Grundkörpers (3) der ersten, tibialen Komponente (2) angeordnet ist.

7. Gelenkprothese nach Anspruch 6,
**dadurch gekennzeichnet,**
**dass** der Führungsstab (8) die Hülse (7a) zumindest teilweise durchgreift.

8. Gelenkprothese nach einem der Ansprüche 1 bis 3,
**dadurch gekennzeichnet,**
**dass** das elastisch verformbare Element in Form eines Dämpfungsrings (15) ausgebildet ist.

9. Gelenkprothese nach Anspruch 8,
**dadurch gekennzeichnet,**
**dass** der Dämpfungsring (15) O-ringförmig ausgebildet ist.

10. Gelenkprothese nach Anspruch 8 oder 9,
**dadurch gekennzeichnet,**
**dass** der Dämpfungsring (15) in einer proximalen Ausnehmung (27) des Grundkörpers (3) der tibialen Komponente (2) angeordnet ist.

11. Gelenkprothese nach Anspruch 10,
**dadurch gekennzeichnet,**
**dass** der Dämpfungsring (15) an einem kippbar gelagerten Körper (14) angeordnet ist.

12. Gelenkprothese nach Anspruch 11,
**dadurch gekennzeichnet,**
**dass** das elastisch verformbare Element in Form einer spiralig mehrschichtig gerollten Kunststofflage (19) ausgebildet ist.

13. Gelenkprothese nach Anspruch 12,
**dadurch gekennzeichnet,**
**dass** die Kunststofflage (19) mit dem sie durchgreifenden Führungselement (8) einteilig ausgebildet oder an diesem fixiert ist.

14. Gelenkprothese nach einem der Ansprüche 1 bis 3,
**dadurch gekennzeichnet,**
**dass** das elastisch verformbare Element in Form von Schüttgut (23) ausgebildet ist, welche in einer Ausnehmung (6) eines Grundkörpers (3) der tibialen Komponente (2) angeordnet ist.

15. Gelenkprothese nach Anspruch 14,
**dadurch gekennzeichnet,**
**dass** das Schüttgut (23) aus Elastomerkügelchen besteht.

16. Gelenkprothese nach Anspruch 14 oder 6,
**dadurch gekennzeichnet,**
**dass** das Führungselement (8) in dem Schüttgut (23) steckend angeordnet ist.

17. Gelenkprothese nach Anspruch 1 oder 2,
**dadurch gekennzeichnet,**
**dass** das elastisch verformbare Element in Form eines Federelements, insbesondere einer Tellerfeder (16), einer Schraubenfeder (17), eines Faltenbalgs (18), einer Rohrfeder (20) oder zumindest einer Blattfeder (22) ausgebildet ist.

18. Gelenkprothese nach Anspruch 17,
**dadurch gekennzeichnet,**
**dass** die Tellerfeder (16) in einem Grundkörper (3) der tibialen Komponente (2) verankert ist.

19. Gelenkprothese nach Anspruch 18,
**dadurch gekennzeichnet,**
**dass** die Tellerfeder (16) rund, oval, länglich oder sternförmig ausgebildet ist.

20. Gelenkprothese nach Anspruch 18 oder 29,
**dadurch gekennzeichnet,**
**dass** die Tellerfeder (16) mit dem Führungselement (8) einstückig ausgebildet oder fest mit diesem verbunden ist.

21. Gelenkprothese nach Anspruch 17,
**dadurch gekennzeichnet,**
**dass** die Schraubenfeder (17) in einer Ausnehmung (6) eines Grundkörpers (3) der tibialen Komponente (2) angeordnet ist.

22. Gelenkprothese nach Anspruch 21,
**dadurch gekennzeichnet,**
**dass** die Schraubenfeder (17) zumindest teilweise von dem Führungselement (8) oder von einem Stummel (8a) des Führungselements (8) durchgriffen ist.

23. Gelenkprothese nach Anspruch 17,
**dadurch gekennzeichnet,**
**dass** durch den Faltenbalg (18) eine kraftschlüssige Verbindung zwischen einem Grundkörper (3) und der Lagerschale (5) der tibialen Komponente (2) etabliert ist.

24. Gelenkprothese nach Anspruch 17,
**dadurch gekennzeichnet,**
**dass** die Rohrfeder (20) Ausnehmungen (21) aufweist, durch deren Form und Anordnung die Elastizität der Rohrfeder (20) in verschiedene Raumrichtungen einstellbar ist.

25. Gelenkprothese nach Anspruch 17,
**dadurch gekennzeichnet,**
**dass** sich die zumindest eine Blattfeder (22) einerseits in einer Ausnehmung (6) eines Grundkörpers (3) der tibialen Komponente (2) und andererseits an dem Führungselement (8) abstützt.

26. Gelenkprothese nach Anspruch 25,
**dadurch gekennzeichnet,**
**dass** zwei oder mehr Blattfedern (22) vorgesehen sind, deren Anzahl der Anzahl von innenliegenden Ecken (28) der Ausnehmung (6) entspricht.

27. Gelenkprothese nach einem der Ansprüche 1 bis 26,
**dadurch gekennzeichnet,**
**dass** der Führungsstab (8) einen extrusionskörperähnlichen oder mehrkantigen Querschnitt aufweist.

28. Gelenkprothese nach einem der Ansprüche 1 bis 27,
**dadurch gekennzeichnet,**
**dass** der Führungsstab (8) einen kreuzförmigen Querschnitt aufweist.

29. Gelenkprothese nach Anspruch 27 oder 28,
**dadurch gekennzeichnet,**
**dass** der Führungsstab (8) in einem Außenquerschnitt mit einem Innenquerschnitt einer Ausnehmung (25) der Hülse (7a) korrespondierend ausgebildet ist.

30. Gelenkprothese nach Anspruch 29,
**dadurch gekennzeichnet,**
**dass** die Ausnehmung (25) der Hülse (7a) einen kreuzförmigen Innenquerschnitt aufweist.

31. Gelenkprothese nach einem der Ansprüche 28 bis 30,
**dadurch gekennzeichnet,**
**dass** eine Länge der die kreuzförmigen Querschnitte bildenden Formen in medial-lateraler Richtung verschieden zu derjenigen der anterio-posteriorer Richtung ist.

32. Gelenkprothese nach einem der Ansprüche 28 bis 31,
**dadurch gekennzeichnet,**
**dass** eine Länge der Balken des kreuzförmigen Querschnitts des Führungsstabes (8) verschieden, insbesondere kürzer, zu derjenigen der Hülse (7a) ist.

33. Gelenkprothese nach einem der Ansprüche 27 bis 32,
**dadurch gekennzeichnet,**
**dass** der Führungsstab (8) abgerundete Kanten aufweist.

34. Gelenkprothese nach einem der Ansprüche 27 bis 33,
**dadurch gekennzeichnet,**
**dass** der Führungsstab (8) einen Versteifungsstab (28) aufweist.

35. Gelenkprothese nach einem der Ansprüche 28 bis 34,
**dadurch gekennzeichnet,**
**dass** die Hülse (7a) einen ovalen Querschnitt aufweist.

36. Gelenkprothese nach einem der Ansprüche 28 bis 35,
**dadurch gekennzeichnet,**
**dass** die Hülse (7a) zumindest eine weitere Ausnehmung (26) aufweist.

37. Gelenkprothese nach Anspruch 36,
**dadurch gekennzeichnet,**
**dass** die zumindest eine weitere Ausnehmung (26) einen runden, ovalen oder sichelförmigen Querschnitt aufweist.

38. Gelenkprothese nach Anspruch 36 oder 37,
**dadurch gekennzeichnet,**
**dass** die zumindest eine weitere Ausnehmung (26) gegenüber der zentralen Ausnehmung (25) in anterio-posteriorer Richtung orientiert ist.

39. Gelenkprothese nach einem der Ansprüche 1 bis 38,
**dadurch gekennzeichnet,**
**dass** das elastische Element (7a; 15; 19; 23) viskoelastisch ist.

## Claims

1. Joint prosthesis (1), in particular knee-joint prosthesis (1), for forming an articulated joint between a first bone and at least one second bone with a first, in particular tibial component (2) which is at least indirectly connected to the first bone, at least one second component on which the second bone is at least indirectly supported and which is mounted in a mobile manner on at least one bearing shell (5) of the first, tibial component (2),
a guide element (8) through which a mobile connection between the first, tibial component (2) and the bearing shell (5) is formed, and
an elastically deformable element which enables the bearing shell (5) to be deflected relative to the first component (2) out of a starting position and returned to the starting position, and which is disposed in the first component (2) of the joint prosthesis (1),
**characterised in**
**that** the guide element (8) is formed as a guide rod which at a proximal end comprises a connecting body (9) which is engaged with a recess (10) of the bearing shell (5), wherein the guide rod is made of an elastic material.

2. Joint prosthesis according to Claim 1,
**characterised in**
**that** the elastically deformable element can deform more easily in the anterio-posterior direction than in the medial-lateral direction.

3. Joint prosthesis according to Claim 1 or 2,
**characterised in**
**that** the elastically deformable element is in the form of an elastomer element (7a; 15; 19; 23).

4. Joint prosthesis according to any one of Claims 1 to 3,
**characterised in**
**that** the elastically deformable element consists of a polymer, in particular of polyethylene.

5. Joint prosthesis according to any one of Claims 1 to 4,
**characterised in**
**that** the elastic element is in the form of a sleeve (7a).

6. Joint prosthesis according to Claim 5,
**characterised in**
**that** the sleeve (7a) is disposed in a recess (6) of a base body (3) of the first, tibial component (2).

7. Joint prosthesis according to Claim 6,
**characterised in**
**that** the guide rod (8) reaches at least partly through the sleeve (7a).

8. Joint prosthesis according to any one of Claims 1 to 3,
**characterised in**
**that** the elastically deformable element is in the form of a damping ring (15).

9. Joint prosthesis according to Claim 8,
**characterised in**
**that** the damping ring (15) is formed in the shape of an O-ring.

10. Joint prosthesis according to Claim 8 or 9,
**characterised in**
**that** the damping ring (15) is disposed in a proximal recess (27) of the base body (3) of the tibial component (2).

11. Joint prosthesis according to Claim 10,
**characterised in**
**that** the damping ring (15) is disposed on a body (14) which is mounted in a tiltable manner.

12. Joint prosthesis according to Claim 11,
**characterised in**
**that** the elastically deformable element is in the form of a plastics layer (19) which is spirally rolled in multiple layers.

13. Joint prosthesis according to Claim 12,
**characterised in**
**that** the plastics layer (19) is formed in one piece with the guide element (8) reaching through it or is fixed thereto.

14. Joint prosthesis according to any one of Claims 1 to 3,
**characterised in**
**that** the elastically deformable element is in the form of loose material (23) which is disposed in a recess (6) of a base body (3) of the tibial component (2).

15. Joint prosthesis according to Claim 14,
**characterised in**
**that** the loose material (23) consists of small elastomer balls.

16. Joint prosthesis according to Claim 14 or 6,
**characterised in**
**that** the guide element (8) is disposed inserted in the loose material (23).

17. Joint prosthesis according to Claim 1 or 2,
**characterised in**
**that** the elastically deformable element is in the form of a spring element, in particular a Belleville spring (16), a helical spring (17), bellows (18), a tube spring (20) or at least one leaf spring (22).

18. Joint prosthesis according to Claim 17,
**characterised in**
**that** the Belleville spring (16) is anchored in a base body (3) of the tibial component (2).

19. Joint prosthesis according to Claim 18,
**characterised in**
**that** the Belleville spring (16) is round, oval, elongate or star-shaped.

20. Joint prosthesis according to Claim 18 or 29,
**characterised in**
**that** the Belleville spring (16) is formed in one piece with the guide element (8) or is firmly connected thereto.

21. Joint prosthesis according to Claim 17,
**characterised in**
**that** the helical spring (17) is disposed in a recess (6) of a base body (3) of the tibial component (2).

22. Joint prosthesis according to Claim 21,
**characterised in**
**that** the guide element (8) or a stump (8a) of the guide element (8) reaches at least partly through the helical spring (17).

23. Joint prosthesis according to Claim 17,
**characterised in**
**that** a non-positive connection between a base body (3) and the bearing shell (5) of the tibial component (2) is established by the bellows (18).

24. Joint prosthesis according to Claim 17,
**characterised in**
**that** the tube spring (20) comprises recesses (21) through the shape and arrangement of which the elasticity of the tube spring (20) in different directions in space can be adjusted.

25. Joint prosthesis according to Claim 17,
**characterised in**
**that** the at least one leaf spring (22) is supported on one side in a recess (6) of a base body (3) of the tibial component (2) and on the other side at the guide element (8) .

26. Joint prosthesis according to Claim 25,
**characterised in**
**that** two or more leaf springs (22) are provided, the number of which corresponds to the number of internal corners (28) of the recess (6).

27. Joint prosthesis according to any one of Claims 1 to 26,
**characterised in**
**that** the guide rod (8) has a cross section which is similar to an extruded body or polygonal.

28. Joint prosthesis according to any one of Claims 1 to 27,
**characterised in**
**that** the guide rod (8) has a cross-shaped cross section.

29. Joint prosthesis according to Claim 27 or 28,
**characterised in**
**that** the guide rod (8) is formed in an external cross section with an internal cross section corresponding to a recess (25) of the sleeve (7a).

30. Joint prosthesis according to Claim 29,
**characterised in**
**that** the recess (25) of the sleeve (7a) has a cross-shaped internal cross section.

31. Joint prosthesis according to any one of Claims 28 to 30,
**characterised in**
**that** a length of the shapes forming the cross-shaped cross sections is different in the medial-lateral direction to that of the anterio-posterior direction.

32. Joint prosthesis according to any one of Claims 28 to 31,
**characterised in**
**that** a length of the bars of the cross-shaped cross section of the guide rod (8) is different to, in particular shorter than, that of the sleeve (7a).

33. Joint prosthesis according to any one of Claims 27 to 32,
**characterised in**
**that** the guide rod (8) has rounded edges.

34. Joint prosthesis according to any one of Claims 27 to 33,
**characterised in**
**that** the guide rod (8) comprises a reinforcing rod (28).

35. Joint prosthesis according to any one of Claims 28 to 34,
**characterised in**
**that** the sleeve (7a) has an oval cross section.

36. Joint prosthesis according to any one of Claims 28 to 35,
**characterised in**
**that** the sleeve (7a) comprises at least one further recess (26).

37. Joint prosthesis according to Claim 36,
**characterised in**
**that** the at least one further recess (26) has a round, an oval or a crescent-shaped cross section.

38. Joint prosthesis according to Claim 36 or 37,
**characterised in**
**that** the at least one further recess (26) is oriented in the anterio-posterior direction with respect to the central recess (25).

39. Joint prosthesis according to any one of Claims 1 to 38,
**characterised in**
**that** the elastic element (7a; 15; 19; 23) is viscoelastic.

## Revendications

1. Prothèse articulaire (1), en particulier prothèse articulaire du genou (1), pour la configuration d'un assemblage articulé entre un premier os et au moins un deuxième os avec un premier composant, notamment tibial (2), qui est relié au moins indirectement au premier os, au moins d'un deuxième composant, sur lequel s'appuie au moins indirectement le deuxième os et qui est logé de manière mobile sur au moins une coquille de support (5) du premier composant tibial (2),
un élément de guidage (8) grâce auquel est formée une articulation mobile entre le premier composant tibial (2) et la coquille de support (5), et
un élément déformable élastiquement, qui permet une déviation de la coquille de support (5) par rapport au premier composant (2) à partir d'une position de sortie ainsi qu'un mouvement retour dans la position de sortie, et qui est agencé dans le premier composant (2) de la prothèse articulaire (1), **caractérisée en ce que**
l'élément de guidage (8) est configuré comme une tige de guidage, qui comporte à une extrémité proximale un corps de raccordement (9) engrené avec une cavité (10) de la coquille de support (5), la tige de guidage étant fabriquée dans un matériau élastique.

2. Prothèse articulaire selon la revendication 1, **caractérisée en ce que** l'élément déformable élastiquement peut être plus facilement déformé dans le sens antério-postérieur que dans le sens médio-latéral.

3. Prothèse articulaire selon la revendication 1 ou 2, **caractérisée en ce que**
l'élément déformable élastiquement est configuré sous la forme d'un élément en élastomère (7a; 15; 19; 23).

4. Prothèse articulaire selon l'une des revendications 1 à 3, **caractérisée en ce que** l'élément déformable élastiquement se compose d'un polymère, en particulier de polyéthylène.

5. Prothèse articulaire selon l'une des revendications 1 à 4, **caractérisée en ce que** l'élément élastique est configuré en forme d'un manchon (7a).

6. Prothèse articulaire selon la revendication 5, **caractérisée en ce que**
Le manchon (7a) est disposé dans une cavité (6) d'un corps de base (3) du premier composant tibial (2).

7. Prothèse articulaire selon la revendication 6, **caractérisée en ce que**
la tige de guidage (8) traverse au moins partiellement le manchon (7a).

8. Prothèse articulaire selon l'une des revendications 1 à 3, **caractérisée en ce que** l'élément déformable élastiquement est configuré sous la forme d'un anneau amortisseur (15).

9. Prothèse articulaire selon la revendication 8,
**caractérisée en ce que**,
l'anneau amortisseur (15) est configuré comme un joint torique.

10. Prothèse articulaire selon la revendication 8 ou 9,
**caractérisée en ce que**,
l'anneau amortisseur (15) est disposé dans une cavité proximale (27) du corps de base (3) du composant tibial (2).

11. Prothèse articulaire selon la revendication 10,
**caractérisée en ce que**,
l'anneau amortisseur (15) est disposé dans un corps logé de manière à pouvoir basculer (14).

12. Prothèse articulaire selon la revendication 11,
**caractérisée en ce que**,
l'élément déformable élastiquement est configuré sous la forme de plusieurs couches de matière plastique enroulées en spirale (19).

13. Prothèse articulaire selon la revendication 12,
**caractérisée en ce que**,
les couches de matière plastique (19) sont configurées en une pièce avec l'élément de guidage (8) qui la traverse est fixé à celle-ci.

14. Prothèse articulaire selon l'une des revendications 1 à 3, **caractérisée en ce que** l'élément déformable élastiquement est configuré sous la forme d'un matériau en vrac (23) qui est disposé dans une cavité (6) d'un corps de base (3) du composant tibial (2).

15. Prothèse articulaire selon la revendication 14, **caractérisée en ce que**
le matériau en vrac (23) se compose de billes d'élastomère.

16. Prothèse articulaire selon la revendication 14 ou 6,
**caractérisée en ce que**,
l'élément de guidage (8) est introduit à l'intérieur du matériau en vrac (23).

17. Prothèse articulaire selon la revendication 1 ou 2,
**caractérisée en ce que**,
l'élément déformable élastiquement est configuré sous la forme d'un élément à ressort, en particulier d'une rondelle-ressort (16), d'un ressort hélicoïdal (17), d'un soufflet d'intercirculation (18), d'un tube-ressort (20) ou au moins d'un ressort à lames (22).

18. Prothèse articulaire selon la revendication 17,
**caractérisée en ce que**,
la rondelle-ressort (16) est ancrée dans un corps de bas (3) du composant tibial (2).

19. Prothèse articulaire selon la revendication 18,
**caractérisée en ce que**,
la rondelle-ressort (16) est configurée de manière ronde, ovale, oblongue ou en forme d'étoile.

20. Prothèse articulaire selon la revendication 18 ou 29,
**caractérisée en ce que**,
la rondelle-ressort (16) est configurée avec l'élément de guidage (8) de manière monobloc ou est solidement reliée à celui-ci.

21. Prothèse articulaire selon la revendication 17,
**caractérisée en ce que**,
le ressort hélicoïdal (17) est disposé dans une cavité (6) d'un corps de base (3) du composant tibial (2).

22. Prothèse articulaire selon la revendication 21,
**caractérisée en ce que**,
le ressort hélicoïdal (17) est traversé au moins partiellement par l'élément de guidage (8) ou par un tronçon (8a) de l'élément de guidage (8).

23. Prothèse articulaire selon la revendication 17,
**caractérisée en ce que**,
le soufflet d'intercirculation (18) permet de raccorder par ressort d'un corps de base (3) à la coquille de support (5) du composant tibial (2).

24. Prothèse articulaire selon la revendication 17,
**caractérisée en ce que**,
le tube-ressort (20) comporte des cavités (21) dont la forme et la disposition permettent de régler l'élasticité du tube-ressort (20) dans différentes directions dans l'espace.

25. Prothèse articulaire selon la revendication 17,
**caractérisée en ce que**,
au moins un ressort à lames (22) s'appuie d'une part dans une cavité (6) d'un corps de base (3) du composant tibial (2) et d'autre part contre l'élément de guidage (8).

26. Prothèse articulaire selon la revendication 25,
**caractérisée en ce que**,
deux ressorts à lames ou plus (22) sont prévus dont le nombre correspond au nombre de coins (28) placés à l'intérieur de la cavité (6).

27. Prothèse articulaire selon l'une des revendications 1 à 26, **caractérisée en ce que**
la tige de guidage (8) comporte une section transversale ressemblant à un corps d'extrusion ou à une section polygonale.

28. Prothèse articulaire selon l'une des revendications 1 à 27, **caractérisée en ce que**
la tige de guidage (8) comporte une section transversale cruciforme.

29. Prothèse articulaire selon la revendication 27 ou 28,
**caractérisée en ce que**,
la tige de guidage (8) est configurée de manière correspondante dans une section transversale extérieure avec une section transversale intérieure d'une cavité (25) de la gaine (7a).

30. Prothèse articulaire selon la revendication 29,
**caractérisée en ce que**,
la cavité (25) de la gaine (7a) comporte une section transversale intérieure cruciforme.

31. Prothèse articulaire selon l'une des revendications 28 à 30, **caractérisée en ce que** une longueur des formes représentant les sections transversales cruciformes dans le sens médio-latéral est différente de celle du sens antério-postérieur.

32. Prothèse articulaire selon l'une des revendications 28 à 31, **caractérisée en ce que** une longueur des barres de la section transversale cruciforme de la tige de guidage (8) est différente, notamment plus courte, que celle de la gaine (7a).

33. Prothèse articulaire selon l'une des revendications 27 à 32, **caractérisée en ce que** la tige de guidage (8) présente des bords arrondis.

34. Prothèse articulaire selon l'une des revendications 27 à 33, **caractérisée en ce que** la tige de guidage (8) comporte une tige de renfort (28).

35. Prothèse articulaire selon l'une des revendications 28 à 34, **caractérisée en ce que** la gaine (7a) comporte une section transversale ovale.

36. Prothèse articulaire selon l'une des revendications 28 à 35, **caractérisée en ce que** la gaine (7a) comporte au moins une autre cavité (26).

37. Prothèse articulaire selon la revendication 36,
**caractérisée en ce que**,
au moins une autre cavité (26) comporte une section transversale ronde, ovale ou en forme de croissant.

38. Prothèse articulaire selon la revendication 36 ou 37,
**caractérisée en ce que**,
au moins une autre cavité (26) est orientée dans le sens antério-postérieur par rapport à la cavité centrale (25).

39. Prothèse articulaire selon l'une des revendications 1 à 38,
**caractérisée en ce que**,
l'élément élastique (7a ; 15 ; 19 ; 23) est viscoélastique.
